(19) Europäisches Patentamt
European Patent Office
Office européen des brevets

(11) **EP 3 990 032 B1**

(12) **EUROPEAN PATENT SPECIFICATION**

(45) Date of publication and mention
of the grant of the patent:
**06.12.2023 Bulletin 2023/49**

(21) Application number: **20733837.7**

(22) Date of filing: **24.06.2020**

(51) International Patent Classification (IPC):
***A61K 49/22*** *(2006.01)*

(52) Cooperative Patent Classification (CPC):
**A61K 49/223**

(86) International application number:
**PCT/EP2020/067752**

(87) International publication number:
**WO 2020/260420 (30.12.2020 Gazette 2020/53)**

(54) **FREEZE-DRIED COMPOSITION FOR PREPARING CALIBRATED GAS-FILLED MICROVESICLES**

GEFRIERGETROCKNETE ZUSAMMENSETZUNG ZUR HERSTELLUNG VON KALIBRIERTEN GASGEFÜLLTEN MIKROVESIKELN

COMPOSITION LYOPHILISÉE POUR PRÉPARER DES MICROVÉSICULES CALIBRÉES REMPLIES DE GAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2019 EP 19182223**

(43) Date of publication of application:
**04.05.2022 Bulletin 2022/18**

(73) Proprietor: **Bracco Suisse SA**
**6814 Cadempino (CH)**

(72) Inventors:
• **LASSUS, Anne**
**1228 Plan-les-Ouates (CH)**

• **CHERKAOUI, Samir**
**1228 Plan-les-Ouates (CH)**

(74) Representative: **Ravizza, Claudio**
**Bracco Suisse S.A.**
**Intellectual Property Department**
**31, route de la Galaise**
**1228 Plan-les-Ouates (CH)**

(56) References cited:
**WO-A1-2018/041906     WO-A1-2019/170606**
**WO-A2-2004/069284**

**Description**

*Technical field*

The present invention generally relates to the field of ultrasound contrast-agents (USCA). In particular, it relates to a freeze-dried composition according to claim 1, which may be reconstituted for preparing a suspension of gas-filled microvesicles with calibrated size, useful in diagnostic or therapeutic applications. It further relates to the method for the preparation of such freeze-dried composition.

*Background of the invention*

Calibrated-size microvesicles (CMV) are a new generation of gaseous microbubbles having a narrow calibrated and controlled size distribution (mean diameter sizes between 3 and 8 $\mu$m) compared to commercially available polydisperse microbubble ultrasound contrast-agents (USCA). These calibrated-size microbubbles are designed to enhance imaging sensitivity and improve efficiency to deliver drug and gene to specific organs. Calibrated microvesicles can be produced using various techniques: decantation, mechanical filtration, centrifugation, bubble sorting and flow-focusing. Particularly, the flow-focusing technology allows the manufacturing of calibrated microvesicles (typically with a geometric standard deviation (GSD) values between 1.05 and 1.08) in a highly reproducible way at a reasonable production rate (~60 million bubbles per minute), with acceptable concentrations of suspended microvesicles (e.g. between $3 \times 10^8$ CMV/mL and $4 \times 10^8$ CMV/mL) for subsequent uses.

*Ref.1* [WO2018/041906 A1 - BRACCO SUISSE SA] and *Ref.2* [PCT application number PCT/EP2019/055325], both in the name of the Applicant, describe a method for preparing CMV, such as gas-filled microbubbles, in particular by using microfluidic technique. Notwithstanding aqueous suspensions of calibrated microvesicles are highly stable at room temperature for few weeks, this stability may pose some constraints for a pharmaceutical product development, for which longer shelf life is generally desirable. There is thus a need to develop a long-term storage procedure. Freeze-drying, also known as lyophilization, is a complex and challenging process, widely used in the pharmaceutical industry that has the advantage of preserving pharmaceutical products under a dry form over several months. In fact, freeze-dried products display greater storage stability and can be easily shipped. Freeze-drying is also used in the field of gas filled microvesicles for preparing freeze-dried dosage forms which are then reconstituted with an aqueous solvent in the presence of a gas, to form a suspension of gas-filled microvesicles.

*Ref.3* [US2017/080113 A1 - GE Healthcare] describes the preparation of a suspension of microbubbles with adjusted sizes and subsequently lyophilization with a sucrose solution thereof.

*Ref.4* [WO97/29782 A1 - NICOMED IMAGING A/S] teaches that USCA precursors which are stable at room temperature (RT) can be prepared by lyophilization of $C_3F_8$ microbubbles in the presence of a freeze-drying stabilizer, preferably sucrose.

Ref.5 [WO 2004/069284 A2 - BRACCO RESEARCH SA [CH]] discloses a method for preparing a lyophilized matrix starting from an emulsion prepared from an aqueous medium, a phospholipid and a water immiscible organic solvent. Upon reconstitution of said lyophilized matrix, a respective injectable contrast agent is obtained.

Up to now, according to Applicant's knowledge, such technique has not been applied yet for preparing a freeze-dried composition from a suspension of calibrated microvesicles.

As observed by the Applicant, one of the most challenging issue in preparing a freeze-dried composition of calibrated microvesicles is related to the need of avoiding substantial modification of the characteristics of the initial suspension of calibrated microvesicles, such as concentration, monodispersity or geometric standard deviation (GSD) and/or final mean diameter.

The Applicant has now found that such initial characteristics, can be preserved to an acceptable extent after the freeze-drying process by using a suitable freeze-drying protecting component at a suitable concentration.

*Summary of the invention*

In a first aspect, the present invention relates to a freeze-dried composition according to claim 1, which, upon reconstitution with a pharmaceutically acceptable solution in the presence of a biocompatible gas, provides a suspension of calibrated gas-filled microvesicles, wherein said reconstituted suspension of microvesicles have a geometric standard deviation (GSD) value of at least 1.22 or lower.

The freeze-drying protecting component is a polyethylene glycol (PEG).

The reconstituted suspension of calibrated microvesicles is characterized by a GSD of at least 1.22 or lower, preferably of at least 1.21, down to e.g. 1.10.

In an embodiment of the invention, said reconstituted suspension of calibrated microvesicles is characterized by a concentration of at least $2.0 \times 10^8$ CMV/mL, preferably $2.1 \times 10^8$ CMV/mL, more preferably $2.3 \times 10^8$ CMV/mL, up

to $5.5 \times 10^8$ CMV/mL.

According to a further aspect, the invention relates to a method of preparing a freeze-dried composition for the preparation of a reconstituted suspension of calibrated gas-filled microvesicles having a geometric standard deviation (GSD) of 1.22 or lower, according to claim 8.

A further aspect of the invention relates to a freeze-dried composition for preparing a suspension of calibrated gas-filled microvesicles having a geometric standard deviation (GSD) of 1.22 or lower, said freeze-dried composition being obtainable by a process according to claim 11.

A further aspect of the invention relates to a process for the preparation of an injectable contrast agent comprising a suspension of gas-filled microvesicles, wherein said process comprises reconstituting a freeze-dried composition as defined above, with a pharmaceutically acceptable solution in the presence of a biocompatible gas.

*Figures*

Figure 1: Typical particle size distribution representation.
Figure 2: A schematic representation of the core portion of a microfluidic flow-focusing device.
Figure 3: An exemplary schematic drawing of a device useful for the process of the invention technology.
Figure 4: Influence of the concentration of PEG4000 and PEG8000 on the CMV yield after freeze-drying at two different freezing temperatures -40 °C and -60 °C.

*Detailed description of the invention*

The expression "gas-filled microvesicles" generally refers to bubbles of gas bounded, at the gas/liquid interface, by a very thin envelope (film) involving a stabilizing amphiphilic material, typically a phospholipid, disposed at the gas to liquid interface. Said calibrated gas-filled microvesicles are suitable as contrast agents in ultrasound imaging techniques, known as Contrast-Enhanced Ultrasound (CEUS) Imaging, or in therapeutic applications, e.g. in combination with ultrasound mediated drug delivery.

These stabilized gas bubbles (dispersed in a suitable physiological solution) are generally referred to in the art with various terminologies, depending typically from the stabilizing material employed for their preparation; these terms include, for instance, "microspheres", "microbubbles", "microcapsules" or "microballoons", globally referred to here as "gas-filled microvesicles" (or "microvesicles" in short).

The term "calibrated" (when referred to gas-filled microvesicles) specifically refers to microvesicles suspensions with highly calibrated microvesicles (CMV), having different sizes between 3 and 8 μm, characterized by a size distribution having a geometric standard deviation (GSD) of at least 1.2 or lower, preferably of at least 1.1, down to e.g. 1.05.

In this description and claims, the term "calibrated" is used interchangeably with "size-controlled", "monodispersed" or "monosize(d)" microvesicles.

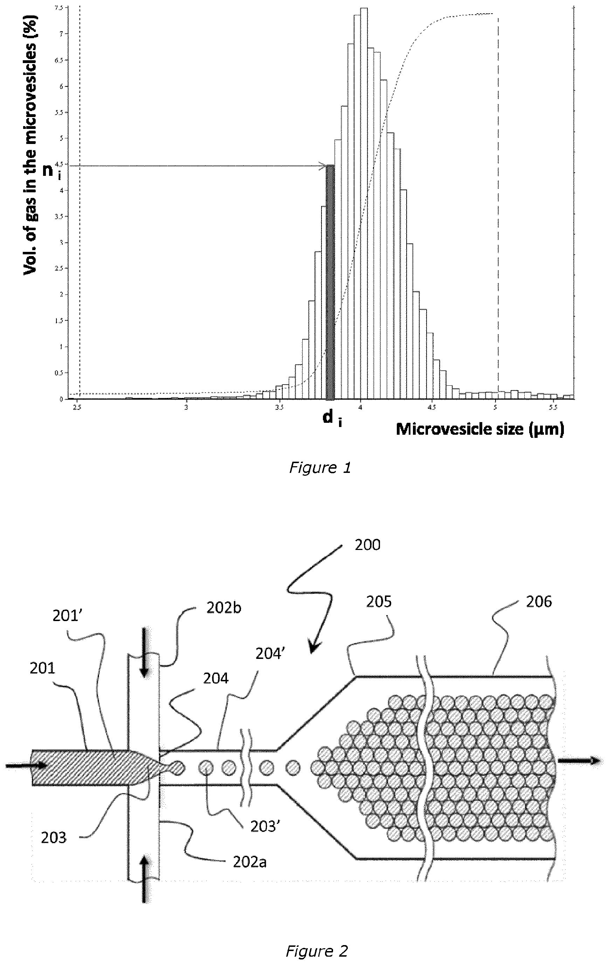

In the present invention, calibrated gas filled microvesicles are preferably produced by using a microfluidic flow-focusing technology, where a gas thread is focused between two liquid flows in a flow-focusing device and phospholipid-stabilized calibrated microvesicles form and are collected in the outlet channel. Through this approach calibrated microvesicles are manufactured in a highly reproducible way at a reasonable production rate (~60 million bubbles per minute) (Fig 1 and Fig.2).

Depending on the parameters of the manufacturing process and device, the calibrated microvesicles may be obtained with relatively narrow size distribution around any desired mean diameter, e.g. from 3 to 8 μm, preferably about 4 μm.

The size distribution of said calibrated microvesicles is typically characterized by a geometric standard deviation (GSD) value of at least 1.20 or lower, preferably of at least 1.15, down to e.g. 1.05.

The calibrated microvesicles concentration (particularly upon production with microfluidic flow-focusing) is typically comprised between $3 \times 10^8$ and $4 \times 10^8$ CMV/mL, preferably close to $4 \times 10^8$ CMV/mL, not lower than $3 \times 10^8$ CMV/mL.

The "geometric standard deviation" (GSD) generally provides a suitable value for characterizing the breath of the size distribution in a population of particles (gas-filled microvesicles in the specific case). A population of particles with a broad range of sizes will thus have a larger GSD value than one in which the particles sizes are narrowly distributed around a mean value (i.e. relatively similar in size).

**Figure 1** shows an example of a size distribution graph (by volume) of a population of gas filled microvesicles which can be obtained with a commercial particle analyser instrument (e.g. Coulter Counter Multisizer 3, equipped with the Multisizer 3 software), by determining the volume of gas for each of its channels, each channel corresponding to a predetermined diameter of the microvesicles (e.g. with increments of 0.1 microns). By the determination of the number of calibrated gas-filled microvesicles in the suspension, their respective diameter and volume distribution in a selected size range (e.g. between 3 $\mu$m and 6 $\mu$m for a 4.5 $\mu$m CMV mean diameter), it is possible to calculate the GSD of a CMV distribution, by using the following **Equation 1:**

$$\mathbf{Eq.1} \qquad GSD = e^{\sqrt{\frac{\sum\left[n_i(ln\ x_i - \ln \bar{x})^2\right]}{\sum n_i}}}$$

Where:

$n_i$ = percentage of volume of gas (with respect to the total one) entrapped in the microvesicles measured for the ith channel

$x_i$ = volume of the microvesicles in the $i^{th}$ channel, where

$$\mathbf{Eq.\ 1.1.} \qquad x_i = d_i^3 . \pi / 6$$

(di = diameter of the microvesicle in the $i_{th}$ channel center)

$\bar{x}$ = geometric mean of the volume of the microvesicles in the selected range, where:

$$\mathbf{Eq.1.2.} \qquad \bar{x} = 10^{\left[\frac{\sum(n_i . \log x_i)}{\sum n_i}\right]}$$

Among the various commercially available measuring devices, the Coulter Counter Multisizer 3, equipped with the Multisizer 3 software, is capable of calculating and providing such GSD value as defined above.

For instance, a GSD value of 1.2 indicates that about the 50% of CMV are calibrated between 2.5 and 5 $\mu$m, for a mean diameter of 4 $\mu$m; a GSD of 1.05-1.08 (<1.1) indicates that about the 90-95% of CMV have sizes comprised between 2.5 and 5 $\mu$m.

The expression "microvesicles concentration" as used herein refers to the number of CMV in a volume unit, determined using a Coulter Counter apparatus, i.e. number of CMV/mL.

As observed by the Applicant, while said calibrated microvesicles obtained through microfluidic flow-focusing can be stored at room temperature for few weeks without substantial impacts on their main characteristics, after said storage period the characteristics of said microvesicles (e.g. concentration and size distribution) may not be maintained and may progressively deteriorate.

The shelf-life of such suspensions of gas-filled microvesicles is thus relatively short for a pharmaceutical product and there is a need to develop a long-term storage procedure, able to preserve the CMV initial characteristics, such as concentration, GSD and final diameter for longer times, e.g. months or years.

The lyophilization process is a suitable approach to obtain a calibrated microvesicles dry form, with increased stability over time and with preserved initial characteristics.

It has now been surprisingly found that CMV initial characteristics, such as concentration, GSD and final diameter, can be substantially preserved after the freeze-drying process by using suitable freeze-drying protecting components.

In a first aspect, the present invention provides a freeze-dried composition according to claim 1, which upon reconstitution with a suitable aqueous solution in the presence of biocompatible gas, provides a suspension of calibrated gas-filled microvesicles, wherein said microvesicles have a GSD value of at least 1.22 or lower, preferably of at least 1.21, down to e.g. 1.10.

In the present description and claims, the term "freeze-drying" and "lyophilization" are used interchangeably, as well the terms "freeze-dried" and "lyophilized".

*Freeze-dried composition*

As used herein, the expression "freeze-dried composition" indicates any dry dosage form for long-term storage of gas-filled microvesicles formulations obtained through a lyophilization process. Said freeze-dried composition can comprise one or more active ingredient and a freeze-drying protecting component.

The expression "active ingredient" as used herein indicates the microvesicle-stabilizing materials, e.g. the amphiphilic materials, which are comprised in the freeze-dried composition with the freeze-drying protecting components.

*Freeze-drying protecting component*

As used herein, the expression "freeze-drying protecting component" refers to a component suitable for freeze drying, which is included in the microvesicles suspension before the lyophilization process thereof.

The term "freeze-drying protecting component" designates any compound added to protect the active ingredient during any phase of the freeze-drying process. Examples of suitable freeze-drying protecting components are polyethylene glycols (PEG), polyols, saccharides, surfactants, buffers, amino acids, chelating complexes, and inorganic salts.

According to an embodiment of the invention, said freeze-drying protecting component is selected among the group of polymers, polyols and saccharides.

In a preferred embodiment of the invention said freeze-drying protecting component is a polymer, preferably a hydrophilic polymer, more preferably a polyglycol.

In a still more preferred embodiment, said polymer is a polyethylene glycol (PEG).

Polyethylene glycol (PEG) has its standard chemical meaning. The chemical formula of PEG is $HOCH_2(CH_2OCH_2)_mCH_2OH$ where $m$ represents the average number of oxyethylene groups. Typical polyethylene glycols are available in a wide range of average molecular weights, starting from 190-210 g/mol (PEG 200; $m$=4.2) to 7000-9000 g/mol (PEG 8000; $m$=181.4).

According to this description, the expression "molecular weight" indicates the average length of the PEG polymer chains, with a variability of ±10% on the indicated molecular weight.

The freeze-drying protecting component is a PEG having a molecular weight comprised between 2000 and 10000 g/mol, preferably between 4000 and 8000 g/mol.

According to an embodiment, the freeze-drying protecting component is PEG having a molecular weight of 8000 g/mol (±10%). According to another embodiment, the freeze-drying protecting component is PEG having a molecular weight close to 4000 g/mol (±10%).

In an embodiment of the invention, the freeze-drying protecting component is added to the suspension of CMV as a solution having a concentration comprised between 100 mg/mL and 300 mg/mL, preferably 120 mg/mL and 250 mg/mL, more preferred close to 200 mg/mL.

In a still more preferred embodiment, the Applicant has found that it is preferred adding a PEG to the suspension of CMV as a solution having a concentration of 200 mg/mL, to improve the preservation of initial characteristics of the CMV suspension reconstituted after the freeze-drying process.

Another parameter which may be considered in the selection of a freeze-drying protecting component is the viscosity.

As used herein, the term viscosity refers to dynamic viscosity ($\mu$, Pa·s units) which is characterized by the resistance to laminar flow of an incompressible fluid (e.g. the CMV suspension either before the freeze-drying step or reconstituted from the freeze-dried product).

The viscosity of a CMV suspension comprising a freeze-drying protecting component can affect its processing e.g. during its manufacturing, or the administration of the reconstituted suspension of CMV from the freeze-dried composition.

Generally, during the manufacturing process, lower viscosity CMV suspensions are preferred, e.g. to avoid excessive overpressure in the microfluidic flow-focusing device.

From another point of view, reconstituted freeze-dried composition having a lower viscosity can facilitate its administration, especially through the injection routes (e.g. parenteral and intradermal).

The viscosity of a suspension depends on the concentration and molecular weight of its components; in particular, in the present case, on the PEG used as freeze-drying protecting component. Precisely, higher concentrations and/or higher molecular weights of the PEG component increase the viscosity of the CMV suspension.

For instance, solutions comprising PEG4000 typically possesses relatively lower viscosity values (in general, from about one half and one third) with respect to solutions comprising an equal amount of PEG8000.

CMV suspension comprising PEG4000, either at 10% or at 20%, may thus provide some advantages in terms of either processing or administration of the suspension.

In this description and claims, the term "polyol" has its conventional chemical meaning; it indicates any organic compound with more than two hydroxyl functional groups, characterized by the general formula $HOCH_2(CHOH)_nCH_2OH$, where n is an integer from 1 to 6, preferably from 2 to 4. Suitable polyols include erythritol, xylitol, sorbitol, lactitol and mannitol.

In this invention, said polyol is preferably selected from the group of polyols having a carbon chain length from four to six carbons atoms.

The term "saccharide" has its standard meaning in the field of chemistry. Saccharides, also called carbohydrates, are molecular compounds made from just three elements: carbon, hydrogen and oxygen. The simplest saccharides are called monosaccharides and they are the building units for bigger saccharides, such as disaccharides, trisaccharide and polysaccharides.

*Amphiphilic material*

Materials suitable for forming the stabilizing layer of the gas-filled microvesicle (i.e. microvesicle-stabilizing materials) are those known in the art. These preferably include amphiphilic materials.

The term "amphiphilic material" as used herein includes compounds having a molecule with a hydrophilic polar head portion (e. g. a polar or ionic group), capable of interacting with an aqueous medium, and a hydrophobic organic tail portion (e. g. a hydrocarbon chain), capable of interacting with e. g. an organic solvent. These compounds thus generally act as "surface active agents", i.e. compounds which are capable of stabilizing mixtures of otherwise generally immiscible materials, such as mixtures of two immiscible liquids (e. g. water and oil), mixtures of liquids with gases (e. g. gas microbubbles in water) or mixtures of liquids with insoluble particles (e.g. metal nanoparticles in water).

Suitable amphiphilic materials comprise, for instance, phospholipids; lysophospholipids; fatty acids, such as palmitic acid, stearic acid, arachidonic acid or oleic acid; lipids bearing polymers, such as chitin, hyaluronic acid, polyvinylpyrrolidone or polyethylene glycol (PEG), also referred as "pegylated lipids"; lipids bearing sulfonated mono- di-, oligo- or polysaccharides; cholesterol, cholesterol sulfate or cholesterol hemisuccinate; tocopherol hemisuccinate; lipids with ether or ester-linked fatty acids; polymerized lipids; diacetyl phosphate; dicetyl phosphate; ceramides; polyoxyethylene fatty acid esters (such as polyoxyethylene fatty acid stearates), polyoxyethylene fatty alcohols, polyoxyethylene fatty alcohol ethers, polyoxyethylated sorbitan fatty acid esters, glycerol polyethylene glycol ricinoleate, ethoxylated soybean sterols, ethoxylated castor oil or ethylene oxide (EO) and propylene oxide (PO) block copolymers; sterol esters of sugar acids including cholesterol glucuronides, lanosterol glucuronides, 7-dehydrocholesterol glucuronide, ergosterol glucuronide, cholesterol gluconate, lanosterol gluconate, or ergosterol gluconate; esters of sugar acids and alcohols including lauryl glucuronide, stearoyl glucuronide, myristoyl glucuronide, lauryl gluconate, myristoyl gluconate, or stearoyl gluconate; esters of sugars with aliphatic acids including sucrose laurate, fructose laurate, sucrose palmitate, sucrose stearate, glucuronic acid, gluconic acid or polyuronic acid; saponins including sarsasapogenin, smilagenin, hederagenin, oleanolic acid, or digitoxigenin; glycerol or glycerol monoesters with fatty acids, including glycerol monopalmitate, glycerol monostearate, , glycerol monomyristate or glycerol monolaurate,; long chain alcohols including n-decyl alcohol, lauryl alcohol, myristyl alcohol, cetyl alcohol, or n-octadecyl alcohol; 6-(5-cholesten-3(3-yloxy)-1-thio- $\beta$ -D-galactopyranoside; digalactosyldiglyceride; 6-(5-cholesten-3 $\beta$ -yloxy)hexyl-6-amino-6-deoxy-1-thio- $\beta$ -D-galactopyranoside; 6-(5-cholesten-3 $\beta$ -yloxy)hexyl-6-amino-6-deoxyl-1-thio- $\beta$ -D-mannopyranoside; 12-(((7'-diethylaminocoumarin-3-yl)carbonyl)methyl-amino)octadecanoic acid; N-[12-(((7'-diethylaminocoumarin-3-yl)carbonyl)methylamino)-octadecanoyl]-2-aminopalmitic acid; N-succinyldioleylphosphatidylethanolamine; 1,2-dioleyl-sn-glycerol; 1,2-dipalmitoyl-sn-3-succinyl-lycerol; 1,3-dipalmitoyl-2-succinylglycerol; 1-hexadecyl-2-palmitoylglycerophosphoethanolamine or palmitoylhomocysteine; alkylamines or alkylammonium salts, comprising at least one ($C_{10}$-$C_{20}$), preferably ($C_{14}$-$C_{18}$), alkyl chain, such as, for instance, N-stearylamine, N,N'-distearylamine, N-hexadecylamine, N,N'-dihexadecylamine, N-stearylammonium chloride, N,N'-distearylammonium chloride, N-hexadecylammonium chloride, N,N'-dihexadecylammonium chloride, dimethyldioctadecylammonium bromide (DDAB), hexadecyltrimethylammonium bromide (CTAB); tertiary or quaternary ammonium salts comprising one or preferably two ($C_{10}$-$C_{20}$), preferably ($C_{14}$-$C_{18}$), acyl chain linked to the N-atom through a ($C_3$-$C_6$) alkylene bridge, such as, for instance, 1,2-distearoyl-3-trimethylammonium-propane (DSTAP), 1,2-dipalmitoyl-3-trimethylammonium-propane (DPTAP), 1,2-oleoyl-3-trimethylammonium-propane (DOTAP), 1,2-distearoyl-3-dimethylammonium-propane (DSDAP); and mixtures or combinations thereof.

According to this invention, the amphiphilic material is a phospholipid.

The term "phospholipid" is intended to encompass any amphiphilic phospholipidic compound, the molecules of which are capable of forming a stabilizing film of material (typically in the form of a mono-molecular layer) at the gas-water boundary interface in the final microbubbles suspension. Accordingly, these materials are also referred to in the art as" film-forming phospholipids".

Examples of suitable phospholipids include esters of glycerol with one or preferably two (equal or different) residues of fatty acids and with phosphoric acid, wherein the phosphoric acid residue is in turn bound to a hydrophilic group, such as, for instance, choline (phosphatidylcholines - PC), serine (phosphatidylserines - PS), glycerol (phosphatidylglycerols - PG), ethanolamine (phosphatidylethanolamines - PE), inositol (phosphatidylinositol). Esters of phospholipids with only one residue of fatty acid are generally referred to in the art as the "lyso" forms of the phospholipid or "lysophospholipids". Fatty acids residues present in the phospholipids are in general long chain aliphatic acids, typically containing from 12 to 24 carbon atoms, preferably from 14 to 22; the aliphatic chain may contain one or more unsaturations or is preferably completely saturated. Examples of suitable fatty acids included in the phospholipids are, for instance, lauric acid, myristic acid, palmitic acid, stearic acid, arachidic acid, behenic acid, oleic acid, linoleic acid, and linolenic acid. Preferably, saturated fatty acids such as myristic acid, palmitic acid, stearic acid and arachidic acid are employed.

Further examples of phospholipids are phosphatidic acids, i.e. the diesters of glycerol-phosphoric acid with fatty acids; sphingolipids such as sphingomyelins, i.e. those phosphatidylcholine analogues where the residue of glycerol diester with fatty acids is replaced by a ceramide chain; cardiolipins, i.e. the esters of 1,3-diphosphatidylglycerol with a fatty acid; glycolipids such as gangliosides GM1 (or GM2) or cerebrosides; glucolipids; sulfatides and glycosphingolipids.

As used herein, the term "phospholipids" include either naturally occurring, semisynthetic or synthetically prepared products that can be employed either singularly or as mixtures.

Examples of naturally occurring phospholipids are natural lecithins (phosphatidylcholine (PC) derivatives) such as, typically, soya bean or egg yolk lecithins.

Examples of semisynthetic phospholipids are the partially or fully hydrogenated derivatives of the naturally occurring lecithins. Preferred phospholipids are fatty acids diesters of phosphatidylcholine, ethylphosphatidylcholine, phosphatidylglycerol, phosphatidic acid, phosphatidylethanolamine, phosphatidylserine, phosphatidylinositol or of sphingomyelin.

Examples of preferred phospholipids are, for instance, dilauroyl-phosphatidylcholine (DLPC), dimyristoyl-phosphatidylcholine (DMPC), dipalmitoyl-phosphatidylcholine (DPPC), diarachidoyl-phosphatidylcholine (DAPC), distearoyl-phosphatidylcholine (DSPC), dioleoyl-phosphatidylcholine (DOPC), 1,2 Distearoyl-*sn*-glycero-3-Ethylphosphocholine (Ethyl-DSPC), dipentadecanoyl-phosphatidylcholine (DPDPC), 1-myristoyl-2-palmitoyl-phosphatidylcholine (MPPC), 1-palmitoyl-2-myristoyl-phosphatidylcholine (PMPC), 1-palmitoyl-2-stearoyl-phosphatidylcholine (PSPC), 1-stearoyl-2-palmitoyl-phosphatidylcholine (SPPC), 1-palmitoyl-2-oleylphosphatidylcholine (POPC), 1-oleyl-2-palmitoyl-phosphatidylcholine (OPPC), dilauroyl-phosphatidylglycerol (DLPG) and its alkali metal salts, diarachidoylphosphatidyl-glycerol (DAPG) and its alkali metal salts, dimyristoylphosphatidylglycerol (DMPG) and its alkali metal salts, dipalmitoylphosphatidylglycerol (DPPG) and its alkali metal salts, distearoylphosphatidylglycerol (DSPG) and its alkali metal salts, dioleoyl-phosphatidylglycerol (DOPG) and its alkali metal salts, dimyristoyl phosphatidic acid (DMPA) and its alkali metal salts, dipalmitoyl phosphatidic acid (DPPA) and its alkali metal salts, distearoyl phosphatidic acid (DSPA), diarachidoyl-lphosphatidic acid (DAPA) and its alkali metal salts, dimyristoyl-phosphatidylethanolamine (DMPE), dipalmitoylphosphatidylethanolamine (DPPE), distearoyl phosphatidyl-ethanolamine (DSPE), dioleylphosphatidyl-ethanolamine (DOPE), diarachidoyl-phosphatidylethanolamine (DAPE), dilinoleylphosphatidylethanolamine (DLPE), dimyristoyl phosphatidylserine (DMPS), diarachidoyl phosphatidylserine (DAPS), dipalmitoyl phosphatidylserine (DPPS), distearoylphosphatidylserine (DSPS), dioleoylphosphatidylserine (DOPS), dipalmitoyl sphingomyelin (DPSP), and distearoylsphingomyelin (DSSP), dilauroyl-phosphatidylinositol (DLPI), diarachidoylphosphatidylinositol (DAPI), dimyristoylphosphatidylinositol (DMPI), dipalmitoylphosphatidylinositol (DPPI), distearoylphosphatidylinositol (DSPI), dioleoyl-phosphatidylinositol (DOPI).

Suitable phospholipids further include phospholipids modified by linking a hydrophilic polymer, such as polyethyleneglycol (PEG) or polypropyleneglycol (PPG), thereto. Preferred polymer-modified phospholipids include "pegylated phospholipids", i.e. phospholipids bound to a PEG polymer. Examples of pegylated phospholipids are pegylated phosphatidylethanolamines ("PE-PEGs" in brief) i.e. phosphatidylethanolamines where the hydrophilic ethanolamine moiety is linked to a PEG molecule of variable molecular weight (e.g. from 300 to 20000 daltons, preferably from 500 to 5000 daltons), such as DPPE-PEG (or DSPE-PEG, DMPE-PEG, DAPE-PEG or DOPE-PEG). For example, DPPE-PEG2000 refers to DPPE having attached thereto a PEG polymer having a mean average molecular weight of about 2000.

Particularly preferred phospholipids are DAPC, DSPC, DPPC, DMPA, DPPA, DSPA, DMPG, DPPG, DSPG, DMPS, DPPS, DSPS and Ethyl-DSPC. Most preferred are DPPG, DPPS and DSPC.

Mixtures of phospholipids can also be used, such as, for instance, mixtures of DPPE and/or DSPE (including pegylated derivatives), DPPC, DSPC and/or DAPC with DSPS, DPPS, DSPA, DPPA, DSPG, DPPG, Ethyl-DSPC and/or Ethyl-DPPC.

For instance, a mixture of phospholipids may include phosphatidylcholine derivatives, phosphatidic acid derivatives and pegylated phosphatidylethanolamine, e.g. DSPC/DPPA/DPPE-PEG, DPPC/DPPA/DPPE-PEG, DSPC/DPPA/DSPE-PEG, DPPC/DPPA/DSPE-PEG, DAPC/DPPA/DPPE-PEG, DAPC/DPPA/DSPE-PEG, DSPC/DSPA/DPPE-PEG, DPPC/DSPA/DSPE-PEG, DSPC/DSPG/DPPE-PEG, DPPC/DSPG/DSPE-PEG.

According to the present invention, the phospholipid can conveniently be used in admixtures with any of the above listed amphiphilic compounds. Thus, for instance, lipids such as cholesterol, ergosterol, phytosterol, sitosterol, lanosterol, tocopherol, propyl gallate or ascorbyl palmitate, fatty acids such as myristic acid, palmitic acid, stearic acid, arachidic acid and derivatives thereof or butylated hydroxytoluene and/or other non-phospholipid compounds can optionally be added to one or more of the foregoing phospholipids, e.g. in proportions preferably ranging from zero to 50% by weight, more preferably up to 25%. For instance, mixtures of amphiphilic materials comprising phospholipids and fatty acids can advantageously be used, including DSPC/DPPG/palmitic acid, DSPC/DPPE-PEG/palmitic acid, DPPC/DPPE-PEG/palmitic acid, DSPC/DSPE-PEG/palmitic acid, DPPC/DSPE-PEG/palmitic acid, DSPC/DPPE-PEG/stearic acid, DPPC/DPPE-PEG/stearic acid, DSPC/DSPE-PEG/stearic acid or DPPC/DSPE-PEG/stearic acid.

The microvesicles prepared according to the invention may optionally comprise a targeting ligand.

The term "targeting ligand" includes within its meaning any compound, moiety or residue having, or being capable to promote, a targeting activity (e.g. including a selective binding) of the microvesicles of a composition of the invention towards any biological or pathological site within a living body. Targets with which targeting ligand may be associated include tissues such as, for instance, myocardial tissue (including myocardial cells and cardiomyocytes), membranous tissues (including endothelium and epithelium), laminae, connective tissue (including interstitial tissue) or tumors; blood clots; and receptors such as, for instance, cell-surface receptors for peptide hormones, neurotransmitters, antigens, complement fragments, and immunoglobulins and cytoplasmic receptors for steroid hormones.

The targeting ligand may be synthetic, semi-synthetic, or naturally occurring. Materials or substances which may serve as targeting ligands include, for example, but are not limited to proteins, including antibodies, antibody fragments, receptor molecules, receptor binding molecules, glycoproteins and lectins; peptides, including oligopeptides and polypeptides; peptidomimetics; saccharides, including mono and polysaccharides; vitamins; steroids, steroid analogs, hormones, cofactors, bioactive agents and genetic material, including nucleosides, nucleotides and polynucleotides.

The targeting ligand may be an amphiphilic compound per se (which is admixed with the other components of the microvesicle) or a compound bound to an amphiphilic molecule (e.g. a phospholipid) employed for the formation of the microvesicles.

_Gas_

Suitable gases comprise biocompatible fluorinated gases, preferably perfluorinated gases. Fluorinated gases include materials which contain at least one fluorine atom such as, for instance, fluorinated hydrocarbons (organic compounds containing one or more carbon atoms and fluorine); sulfur hexafluoride; fluorinated, preferably perfluorinated, ketones such as perfluoroacetone; and fluorinated, preferably perfluorinated, ethers such as perfluorodiethyl ether. Preferred compounds are perfluorinated gases, such as $SF_6$ or perfluorocarbons (perfluorinated hydrocarbons), i.e. hydrocarbons where all the hydrogen atoms are replaced by fluorine atoms, which are known to form particularly stable gas-filled microvesicles suspensions.

The term "perfluorocarbon" includes saturated, unsaturated, and cyclic perfluorocarbons. Examples of biocompatible, physiologically acceptable perfluorocarbons are: perfluoroalkanes, such as perfluoromethane, perfluoroethane, perfluoropropanes, perfluorobutanes (e.g. perfluoro-n-butane, optionally in admixture with other isomers such as perfluoro-isobutane), perfluoropentanes, perfluorohexanes or perfluoroheptanes; perfluoroalkenes, such as perfluoropropene, perfluorobutenes (e.g. perfluorobut-2ene) or perfluorobutadiene; perfluoroalkynes (e.g. perfluorobut-2-yne); and perfluorocycloalkanes (e.g. perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane and perfluorocycloheptane). Preferred saturated perfluorocarbons include, for example, $CF_4$, $C_2F_6$, $C_3F_8$, $C_4F_8$, $C_4F_{10}$, $C_5F_{12}$ and $C_6F_{14}$. Particularly preferred gases are those which are in gaseous form at room temperature, including $SF_6$, $C_3F_8$ and $C_4F_{10}$.

An embodiment thus relates to a method of preparing a freeze-dried composition for long term storage of calibrated gas-filled microvesicles, comprising the steps of:

a. preparing a suspension of calibrated gas-filled microvesicles comprising a polyglycol as a freeze-drying protecting component;
b. freeze-drying the calibrated microvesicles suspension.

Preferably, the method of preparation of step a) is the microfluidic flow-focusing technique, as illustrated in _Ref.1_ [WO2018/041906 A1 - BRACCO SUISSE SA] and _Ref.2_ [PCT application number PCT/EP2019/055325].

**Figure 2** shows a schematic representation of the core portion **200** of a flow-focusing device ("microfluidic chip") useful in the process of the invention. The chip comprises a first feed channel **201** for feeding the gaseous flow **201'** and two additional orthogonal feed channels **202a** and **202b** for supplying the liquid flow comprising the amphiphilic material.

The gas flow and the two liquid flows are directed towards the contact zone **203** and then through the calibrated orifice **204,** shown as a dotted line in **Figure 2**. The calibrated orifice is connected to a calibrated channel **204'** having preferably the same cross-section as the orifice, which is in turn connected to an initial portion **205** of the outlet channel **206**. In an alternative embodiment (not shown) the calibrated orifice **204** may be a nozzle directly connected to the initial portion **205** of outlet channel **206** i.e. without the calibrated channel in-between. The microvesicles **203'** are formed in the calibrated orifice and directed, through calibrated channel **204'**, to the initial portion **205** of the outlet channel **206**. The hydraulic diameter of the outlet channel is generally larger than the hydraulic diameter of the calibrated orifice and typically increases from the initial diameter of the calibrated orifice to the final diameter of the outlet channel **206,** corresponding substantially to the hydraulic diameter of a collecting tube (not shown), connecting the flow-focusing device to a container, e.g. a sealed vial for collecting the suspension of microvesicles.

In the initial portion **205** of the outlet channel of the device and preferably also in the contact zone **203** and in the calibrated orifice **204** the temperature of the microvesicles is controlled, as described in _Ref.1_ [WO2018/041906 A1 - BRACCO SUISSE SA] and _Ref.2_ [PCT application number PCT/EP2019/055325].

*Liquid flow*

The aqueous liquid flow for preparing the calibrated gas-filled microvesicles according to the method of the invention comprises an amphiphilic material (as above defined) at a concentration of e.g. from 5.0 to 20 mg/mL, preferably from 7.5 to 15 mg/mL, dispersed in an aqueous carrier.

Suitable aqueous carriers, which are preferably physiologically acceptable, comprise water (preferably sterile water), aqueous solutions such as saline (which may advantageously be balanced so that the final product for injection is not hypotonic), or solutions of one or more tonicity adjusting substances. Tonicity adjusting substances comprise salts or sugars, sugar alcohols, glycols or other non-ionic polyol materials (e.g. glucose, sucrose, sorbitol, mannitol, glycerol, polyethylene glycols, propylene glycols and the like), chitosan derivatives, such as carboxymethyl chitosan, trimethyl chitosan or gelifying compounds, such as carboxymethylcellulose, hydroxyethyl starch or dextran.

In an alternative embodiment, an additional oil phase may be added for incorporating therapeutic hydrophobic substances into the microvesicles. To this end, two additional conduits may be provided in the device for supplying the desired oil phase, as described for instance by Ref.1 [WO2018/041906 A1 - BRACCO SUISSE SA] and Ref.2 [PCT application number PCT/EP2019/055325]. The formed gas-filled microvesicles will thus have a film of oil disposed at the interface between gas and the stabilizing layer of amphiphilic material, which can be loaded with a desired therapeutic agent. Suitable oils may include any biocompatible oil which is liquid at room temperature including, for instance, mono-, di- or tri-esters of glycerol with saturated or unsaturated ($C_2$-$C_{18}$) alkyl chains (including homo- or hetero-allkylesters), such as glycerol monobutyrin, glycerol monolinoleate, 1,2-dihexanoyl glycerol, 1,2 dioctanoyl glycerol, 1,2-dioleyl-sn-glycerol, triacetin, tributyrin, tricaproin, tricaprylin, tricaprin, and mixtures thereof; or natural oils such as soya oil, olive oil, safflower seed oil, sunflower seed oil, peanut oil and mixtures thereof.

*Mixed gas flow*

The freshly formed microvesicles comprise a gas selected among those previously indicated. Preferably the gas is a mixture of a gas highly soluble in water ("HS gas") and of a gas with low solubility in water ("LS gas"), as anticipated in *Ref.* 2 [PCT application number PCT/EP2019/055325].

During the stabilization phase, the major amount of the highly soluble gas rapidly dissolves in water while the poorly soluble one remains entrapped into the densely packed layer of amphiphilic compounds, typically with some residual amount of HS solubility gas dispersed therein.

Examples of HS gases include nitrogen, air, and carbon dioxide, this latter being particularly preferred because of its higher solubility in water.

Suitable LS gases are fluorinated gases, preferably perfluorinated gases. Fluorinated gases those previously described in the present description.

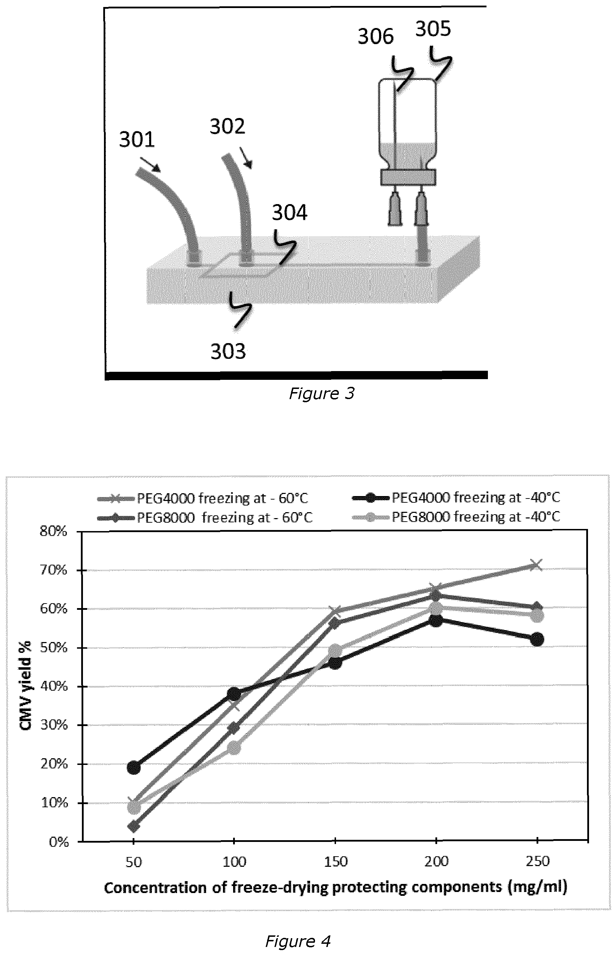

In a preferred embodiment of the invention, gas-filled microvesicles comprising $CO_2$/$C_4F_{10}$ in a volume ratio of from 80/20 to 90/10, e.g. 85/15 can be prepared with a gas-mixing device similar to the one schematically illustrated in **Figure 3.**

**Figure 3** shows an example of a microfluidic flow-focusing device used to produce calibrated microvesicles. A gas flow **302** (comprising e.g. a mixture of $C_4F_{10}$ and $CO_2$) and a liquid flow **301** (comprising an amphiphilic material, e.g. a phospholipid, fatty acid or mixtures thereof), are supplied to microfluidic chip **303** to produce microvesicles through orifice **304.** The microvesicles suspension is collected in a vial **305,** which is preferably prefilled with a gas (e.g. $C_4F_{10}$) at ambient pressure. A venting device (e.g. a needle **306**) is preferably used to equalize the overpressure generated by the liquid filling of the vial. At the end of the collection of the microvesicles suspension, the venting device is preferably removed and the container is preferably sealed to avoid further gaseous exchange with the external atmosphere.

According to a preferred embodiment of this invention, after the collection phase, the calibrated microvesicles obtained through the microfluidic flow-focusing method, are treated using suitable washing techniques, in order to remove not-assembled amphiphilic material and possible residue compounds.

In the present description, the term "washing" indicates any operation carried out on the freshly prepared microvesicles suspension, finalized to remove (or substantially reduce the amount of) not-assembled amphiphilic material and residue compounds.

According to this description, suitable washing techniques comprise centrifugation, filtration, bubble sorting and decantation.

In the present description, the expression "not-assembled amphiphilic material" indicates amphiphilic molecules that, at the end of the preparation process, are present in the calibrated microvesicles suspension, but are not forming the stabilizing layer of the gas-filled microvesicles.

In the present description "residues compounds" indicate any possible additive substance that is added to the amphiphilic material solution during the microvesicles preparation, such as tonicity adjusters as described before.

In a preferred embodiment of this invention, after the washing operation, a freeze-drying protecting component is added to the calibrated microvesicles suspension.

Alternatively, the freeze-drying protecting component can be added to the liquid flow comprising the amphiphilic compounds, described above, during the preparation of the microvesicles by microfluidic technique.

Initial CMV characteristics are particularly preserved when using a polyethyleneglycol as freeze-drying protecting component characterized in that said polyethyleneglycol is added to the suspension of CMV as a solution having a total concentration comprised between 100 mg/ml and 300 mg/mL, preferably between 120 mg/ml and 250 mg/mL, more preferably the total concentration of PEG is 200 mg/mL.

In a preferred embodiment of the invention, before the freeze-drying process, the CMV suspension comprises a polyethyleneglycol as freeze-drying protecting component at a oncentration between 12 and 25%, preferably between 14 and 24% and still more preferred between 18-22% (w/v%).

The freeze-drying protecting component represents the larger amount of the final freeze-dried preparation, wherein it is typically at least 90%, preferably between 94% and 99.7%, more preferably 99,5%, up to 99.9% (w/w).

Said freeze-drying protecting components are those described above in the present description. Using polyethylene glycols as freeze-drying protecting components has shown advantageous results when used in the freeze-drying process of the calibrated microvesicles suspensions, to prepare a freeze-dried composition which can then be reconstituted to obtain suspensions of calibrated microvesicles having acceptable characteristics in terms of concentration and size distribution, as compared to those of the initial suspension (before freeze-drying).

For instance, the addition of a polyethylene glycol as protecting component results to be the best strategy to substantially preserve the GSD value after the freeze-drying process when compared with the addition of a polyol or a saccharide. According to an embodiment, GSD values comprised between 1.20 and 1.22 can be obtained when using a polyethylene glycol, in comparison with the use of a polyol or a saccharide which gives calibrated microvesicles characterized by a higher GSD value, up to 1.34.

In a preferred embodiment, the use of a polyethylene glycol as freeze-drying protecting component is able to significantly improve the yield of calibrated microvesicles after reconstitution of the freeze-dried product with values higher than 60%, e.g 68%, higher than those obtained with the use of a polyol or a saccharide.

In this description and claims, the term freeze-drying has its standard meaning in the pharmaceutical technology field. Freeze-drying process consists of drying a pre-frozen liquid product under low pressure or vacuum and at low temperature. The main objective is to remove liquid from the product in order to provide a freeze-dried product suitable for long term storage.

As observed by the Applicant, freeze-drying parameters may be selected to further optimize the characteristics of the reconstituted suspension of microvesicles (e.g. yield, size, GSD).

In a preferred embodiment of the invention, the freezing temperature of said method for long term storage of calibrated gas-filled microvesicles, ranges between -30 °C and - 70 °C, preferably between -30 °C and -60 °C.

In a still preferred embodiment, the negative pressure applied during the freeze-drying process is preferably of 0.5 mbar or lower, preferably 0.2 or lower, e.g. close to 0.1 mbar.

A further aspect of the invention relates to a freeze-dried composition for preparing a suspension of calibrated gas-filled microvesicles having a geometric standard deviation (GSD) of 1.22 or lower, said freeze-dried composition being obtainable by a process according to claim 11.

A further aspect of the invention relates to a process for the preparation of an injectable contrast agent comprising a suspension of gas-filled microvesicles, wherein said process comprises reconstituting a freeze-dried composition, obtained as described above, comprising an amphiphilic material and a freeze-drying protecting component, with a pharmaceutically acceptable solution in the presence of biocompatible gas.

The freeze-dried composition can then be reconstituted with a suitable pharmaceutically acceptable (aqueous) solution in the presence of biocompatible gas, thus providing a suspension of calibrated gas-filled microvesicles, wherein said microvesicles have a GSD of at least 1.22 or lower, preferably of at least 1.21, down to e.g. 1.10.

In this invention, pharmaceutically acceptable (aqueous) solutions are water, typically sterile, pyrogen free water (to prevent as much as possible contamination in the final reconstituted product), aqueous solutions such as saline (which may advantageously be balanced so that the final product for injection is not hypotonic), or aqueous solutions of one or more tonicity adjusting substances such as salts or sugars, sugar alcohols, glycols or other non-ionic polyol materials.

The freeze-dried composition is typically reconstituted with a volume of aqueous solution similar to the volume of suspension which underwent the freeze-drying process. Accordingly, the concentration of the freeze-drying protecting components in the reconstituted suspension is substantially the same as the one in the initial suspension.

Another embodiment of the invention thus relates to a reconstituted CMV suspension having a GSD of at least 1.22 and a polyethylene glycol at a concentration between 12 and 25%, preferably between 14 and 24% and still more preferred between 18-22%.

These amounts of freeze-drying protecting components are typically higher than the amounts in prior art preparations of gas-filled microvesicles, which are generally lower than 10% (w/w).

Surprisingly, the reconstituted suspensions of calibrated microvesicles were found to have substantially maintained the initial characteristics of calibrated microvesicles, as characterized before the lyophilization process, and thus suitable for subsequent pharmaceutical uses.

In an embodiment of this invention, said reconstituted suspension of calibrated microvesicles is characterized by a concentration of at least $2.0 \times 10^8$ CMV/mL, preferably $2.1 \times 10^8$ CMV/mL, more preferably $2.3 \times 10^8$ CMV/mL, up to $5.5 \times 10^8$ CMV/mL.

As indicated above, the expression "microvesicles concentration" refers to the number of microvesicles in a volume unit, determined using a Coulter Counter apparatus, i.e. number of CMV/mL.

Typically, the concentration of calibrated microvesicles (%), measured after the reconstitution of the freeze-dried composition of the invention with suitable aqueous solution, allows to determine the microbubble yield after said reconstitution, in comparison to the microvesicles concentration measured before the freeze-drying process.

In the present invention, the calibrated microvesicles yield after the reconstitution of the freeze-dried composition of the invention is at least 50%, preferably at least 55%, more preferably at least 60% even more preferably at least 65%, up to e.g. 85%, preferably 90%, more preferably 95%, even more preferably 100%.

The expression "calibrated microvesicles yield" refers to the ratio between the microvesicles concentration measured before freeze-drying and the microvesicles concentration measured after reconstitution of the freeze-dried product with physiologically acceptable solution (in short "after freeze-drying") (see **Equation 2**):

$$\text{Eq.2: CMV yield after freeze-drying (\%)} = (\text{CMV concentration after freeze-drying /mL}) / (\text{CMV concentration before freeze-drying /mL})$$

*Use*

The microvesicles prepared according to the method of the invention may be used in a variety of diagnostic and/or therapeutic techniques, including in particular Ultrasound and Magnetic Resonance.

Diagnostic methods include any method where the use of the gas-filled microvesicles allows enhancing the visualisation of a portion or of a part of an animal (including humans) body, including imaging for preclinical and clinical research purposes. A variety of imaging techniques may be employed in ultrasound applications, for example including fundamental and harmonic B-mode imaging, pulse or phase inversion imaging and fundamental and harmonic Doppler imaging; if desired three-dimensional imaging techniques may be used.

Microvesicles according to the invention may typically be administered in a concentration of from about 0.01 to about 1.0 $\mu$L of gas per kg of patient, depending e.g. on their respective composition, the tissue or organ to be imaged and/or the chosen imaging technique. This general concentration range may of course vary depending on specific imaging applications, e.g. when signals can be observed at very low doses such as in colour Doppler or power pulse inversion.

In an embodiment said method of diagnosing comprises:

(i) administering to a patient a suspension of gas-filled microvesicles obtained by reconstitution of a freeze-dried product obtained according to the process of the invention; and
(ii) detecting an ultrasound signal from a region of interest in said patient.

According to an embodiment, said suspension of gas-filled microvesicles comprises an amphiphilic material and a freeze-drying protecting component.

Reconstitution of the freeze-dried product is preferably made by dispersing it into a physiologically acceptable aqueous carrier, e.g. saline, in the presence of a physiologically acceptable gas, e.g $C_4F_{10}$, under gentle agitation.

Possible other diagnostic imaging applications include scintigraphy, light imaging, and X-ray imaging, including X-ray phase contrast imaging.

Another aspect of the invention relates to the use in a method of therapeutic treatment of a suspension of microvesicles reconstituted from freeze-dried product according to the invention.

Therapeutic techniques include any method of treatment (as above defined) of a patient which comprises the combined use of ultrasounds and gas-filled microvesicles either as such (e.g. in ultrasound mediated thrombolysis, high intensity focused ultrasound ablation, blood-brain barrier permeabilization, immunomodulation, neuromudulation, radiosensitization) or in combination with a therapeutic agent (i.e. ultrasound mediated delivery, e.g. for the delivery of a drug or bioactive compound to a selected site or tissue, such as in tumor treatment, gene therapy, infectious diseases therapy, metabolic diseases therapy, chronic diseases therapy, degenerative diseases therapy, inflammatory diseases therapy, immunologic or autoimmune diseases therapy or in the use as vaccine), whereby the presence of the gas-filled microvesicles may provide a therapeutic effect itself or is capable of enhancing the therapeutic effects of the applied ultrasounds, e.g. by exerting or being responsible to exert a biological effect in vitro and/or in vivo, either by itself or upon specific activation by various physical methods (including e.g. ultrasound mediated delivery).

Microvesicles according to the invention can typically be administered for therapeutic purposes in a concentration of from about 0.01 to about 5.0 $\mu$L of gas per kg of patient, depending e.g. from their respective composition, the type of subject under treatment, the tissue or organ to be treated and/or the therapeutic method applied.

In an embodiment said method of ultrasound therapeutic treatment comprises:

(i) administering to a patient a suspension of gas-filled microvesicles obtained by reconstitution of a freeze-dried product obtained according to the process of the invention;

(ii) identifying a region of interest in said patient to be submitted to a therapeutic treatment, said region of interest comprising said suspension of gas-filled microvesicles; and

(iii) applying an ultrasound beam for therapeutically treating said region of interest;

whereby said ultrasound therapeutic treatment is enhanced by the presence of said suspension of gas-filled microvesicles in said region of interest.

The following examples will help to further illustrate the invention.

### Examples

### Example 1

### Preparation of gas-filled microvesicles

Gas-microvesicles were synthesized using a commercially available microfluidic flow-focusing device (CU4553.007 N30 design, Micronit Microfluidics, NL), mounted in a commercially available chip holder (Micronit microfluidics, Fluidic Connect PRO Chip Holder with 4515 Inserts). The microvesicles formation channel had a width of 19 $\mu$m. The chip and its holder were positioned in an optically transparent temperature controlled water bath that was mounted on an inverted microscope equipped with a 20 times magnification objective (Olympus, LMPLAN 20x) and a CCD camera (Lumenera, LM156M). The temperature of the thermostatic bath was set at 50 °C.

The amphiphilic materials in the liquid flow were: DSPC:DPPE-PEG5000 in a respective molar ratio of 9:1.

The materials were added with the above molar ratios at a concentration of 20 mg/mL to a 2:1 (volume ratio) chloroform/methanol mixture under stirring at 60 °C until complete dissolution the amphiphilic material. The solvent was then evaporated under reduced pressure and the obtained film was dried overnight under reduced pressure. The dried material was then redispersed (at concentrations of 15 mg/mL) in saline (0.9% NaCl) at 60 °C under stirring for 30 minutes. The dispersion was then sonicated by using a tip sonicator (Branson Sonifier 250) to homogenously disperse the material. The preparations were then filtered using a polycarbonate filter (0.45 $\mu$m pore size), cooled down to room temperature and degassed.

Gas-filled microvesicles comprising $CO_2/C_4F_{10}$ in a volume ratio of 85/15 were prepared with a gas-mixing device similar to the one schematically illustrated in **Figure 3**. Briefly, two gas containers were filled with $CO_2$ and $C_4F_{10}$, respectively. The gas flow of each gas was regulated by respective mass flow controllers: (i) EL-Flow: F200CV-002-RAD-11-K, for the $CO_2$ and (ii) Low-$\Delta$P-Flow: F-200DV-RAD-11-Z for $C_4F_{10}$ (both gas controllers from Bronkhorst, Ruurlo, The Netherlands). The mass flow controllers were controlled by a customized software program implemented in Matlab (Mathworks), which was installed on a personal computer, in order to set and keep the desired mixing ratio. A pressure sensor (PSE530-M5-L; SMC Corp., Tokyo, Japan) measured the actual pressure in the gas mixture in the outlet channel leading to the microfluidic chip; a gas pressure of 2 bars was used for the formation of the microvesicles. The liquid co-flow rate was controlled by using a separate mass flow controller (Mini Cori Flow: M13V14I-MAD-11-K-S; Bronkhorst, Ruurlo, The Netherlands). A liquid co-flow rate of around 150 $\mu$L/min was used to operate the flow-focusing device in the jetting regime and produce microvesicles with a diameter (mode) of around 4 $\mu$m.

### Example 2

### Preparation of the freeze-dried composition

Firstly, 3 mL of the calibrated microvesicles suspension obtained through the microfluidic flow-focusing method, as described in Example 1, were transferred in a Pyrex tube (Pyrex disposable tube 12 $\times$ 75 mm) without further addition of $C_4F_{10}$ in the tube. Then, the calibrated microvesicles suspension was centrifuged (Sigma 3-16 Centrifuge) for 6 minutes at 64 g, and the infranatant was withdrawn by means of a syringe equipped with a needle. The remaining 200 $\mu$L of washed microvesicles were redispersed with 3 mL of a solution comprising the freeze-drying protecting component as detailed in the following examples.

The calibrated microvesicles suspended in the solution of the freeze-drying protecting component were then aliquoted in DIN8R glass vials (1.5 mL suspension/vial) and transferred in the freeze dryer.

The vials were cooled at temperatures between -30 °C and -60 °C (as detailed in the subsequent examples) and freeze-dried for approximately 1 hour under vacuum. At the end of the procedure, a freeze-dried composition was obtained as a white homogenous dry solid. The head space was then filled with pure $C_4F_{10}$.

*Example 3*

*Effect of the freeze-drying protecting components on calibrated microvesicles characteristics*

**Table 2** lists the freeze-drying protecting components which were investigated.

**Table 2** Selected freeze-drying protecting components, classified by chemical class

| Molecule | Class | Molecular Weight (g/mol) |
|---|---|---|
| PEG4000 | Polymer | 4'000 |
| PEG8000 | Polymer | 8'000 |
| Xylitol | Polyol | 152.2 |
| Sorbitol | Polyol | 182.2 |
| Mannitol | Polyol | 182.2 |
| Glucose | Monosaccharide | 180.2 |
| Maltose | Disaccharide | 342.3 |
| Sucrose | Disaccharide | 342.3 |
| Dextran 6000 | Polysaccharide | 6'000 |

The above materials were used, at various concentrations, in the preparation of the freeze-dried composition illustrated in Example 2 at a freezing temperature of -60 °C. **Table 3** illustrates the composition and concentration of the investigated freeze-dried protecting components (1A, 1B, 2A, 2B, S3-S13).

**Table 3** Composition and concentration of the evaluated solutions of freeze-drying protecting components

| Prep No. | Freeze-drying protecting components | Total concentration of the freeze-drying protecting components (mg/mL) |
|---|---|---|
| 1A | PEG4000 | 100 |
| 1B | PEG4000 | 200 |
| 2A | PEG8000 | 100 |
| 2B | PEG8000 | 200 |
| S3A | Sorbitol | 100 |
| S3B | Sorbitol | 200 |
| S4 | Xylitol | 100 |
| S5 | Mannitol | 100 |
| S6 | Sucrose | 100 |
| S7A | Maltose | 100 |
| S7B | Maltose | 200 |
| S8 | Raffinose | 100 |
| S9 | Dextran 6000 | 100 |

Characterization

After the freeze-drying process, each freeze-dried composition was reconstituted with 1.5 mL of aqueous solution in the presence of a biocompatible gas, in order to obtain a calibrated and stable microvesicles suspension. The freeze-dried composition was thus reconstituted with a volume of aqueous solution similar to the volume of suspension which underwent the freeze-drying process. Accordingly, the obtained concentrations of the freeze-drying protecting components in the reconstituted suspension were 93,75 mg/mL and 188 mg/mL, respectively starting from a solution at a concentration of 100 mg/mL and 200 mg/mL.

After reconstitution, the reconstituted calibrated microvesicles suspensions were let 5 min on the bench before being characterized using a Coulter Counter Multisizer 3 fitted with a 30 $\mu$m aperture tube, to measure the size, the geometric standard deviation (GSD), the concentration of microvesicles and the yield after the freeze-drying process.

Results

The main results of the characterization of the reconstituted calibrated microvesicles suspensions are reported in **Table 4.**

The GSD value and the concentration of microvesicles were measured before and after the freeze-drying process to evaluate the efficacy of the freeze-drying protecting components in preserving the initial characteristics of the calibrated microvesicles.

**Table 4** GSD, CMV yield values and concentration of calibrated vesicles suspensions obtained after reconstitution of lyophilized compositions comprising a single freeze-drying protecting component (FD= freeze-drying).

| Prep No. | Total concentration of the freeze-drying protecting component (mg/mL) | GSD | CMV Yield after FD (2-6 $\mu$m) (%) | Concentration (CMV/mL) |
|---|---|---|---|---|
| 1A | 100 | 1.20 | 39 | $1.6\times10^8$ |
| 1B | 200 | 1.22 | 63 | $2.3\times10^8$ |
| 2A | 100 | 1.21 | 26 | $0.9\times10^8$ |
| 2B | 200 | 1.20 | 64 | $2.0\times10^8$ |
| S3A | 100 | 1.33 | 3 | $0.1\times10^8$ |
| S3B | 200 | 1.25 | 5 | $0.2\times10^8$ |
| S4 | 100 | 1.34 | 8 | $0.3\times10^8$ |
| S5 | 100 | 1.34 | 14 | $0.5\times10^8$ |
| S6 | 100 | 1.28 | 13 | $0.4\times10^8$ |
| S7A | 100 | 1.28 | 22 | $1.0\times10^8$ |
| S7B | 200 | 1.27 | 21 | $0.9\times10^8$ |
| S8 | 100 | 1.22 | 23 | $0.9\times10^8$ |
| S9 | 100 | 1.26 | 21 | $0.8\times10^8$ |

Considering the GSD value and the microvesicles yield after freeze-drying, results clearly showed that the addition of polyethylene glycols to the calibrated microvesicles suspension, before the freeze-drying process, is much more effective than the addition of polyols or saccharides in term of preservation of the microvesicles characteristics.

In particular, freeze-drying of calibrated microvesicles in polyethylene glycols (PEG4000 and PEG8000) solutions enabled to improve the GSD values and/or the microvesicles yield.

Furthermore, the addition of polyethylene glycols solutions at a concentration of 200 mg/mL to the CMV suspension gave better results than adding polyethylene glycols at a concentration of 100 mg/mL. In particular, the CMV suspensions containing PEG4000 at a concentration of about 200 mg/mL (1B) were characterized by a GSD of 1.22 and a remarkable CMV yield of 63%. Still more advantageously, using a solution of PEG8000 at 200 mg/mL (2B) allowed to reach a GSD of 1.20 and CMV yield of 64%.

*Example 4*

*Characterization of the calibrated microvesicles characteristics after* freeze-drying *at different freezing temperatures.*

The preparation of the freeze-dried composition was further investigated by evaluating different freezing temperatures at which the freshly-prepared calibrated microvesicles are cooled at the beginning of the lyophilization procedure.

The preparation process was performed as previously described in Example 2, except that the vials were cooled at different freezing temperatures, as reported in **Table 5.**

At the end of the freeze-drying process, each lyophilized composition was reconstituted with 1.5 mL of aqueous solution in the presence of a biocompatible gas, in order to obtain a calibrated and stable microvesicles suspension. The freeze-dried composition was thus reconstituted with a volume of aqueous solution similar to the volume of suspension which underwent the freeze-drying process. Accordingly, the obtained concentrations of the freeze-drying protecting components in the reconstituted suspension were 93,75 mg/mL and 188 mg/mL, respectively starting from a solution of freeze-drying protecting components at a concentration of 100 mg/mL and 200 mg/mL.

After reconstitution, the reconstituted calibrated microvesicles suspensions were let 5 min on the bench before being characterized using a Coulter Counter Multisizer 3 fitted with a 30 $\mu$m aperture tube, to measure the size, the geometric standard deviation (GSD), the concentration of microvesicles and the yield after the freeze-drying process.

**Table 5** reports the GSD values and the CMV yield of the microvesicles suspensions, obtained after reconstitution of lyophilized compositions comprising different freeze-drying protecting components. Formulations 2A, 2B and S3A were selected and different freezing temperatures were tested.

Results

Results confirmed that the addition of PEG8000 to the calibrated microvesicles suspension allowed to improve the freeze-drying performance at any investigated freezing temperature, when compared with the use of sorbitol. For instance, the use of PEG800O at 100 mg/mL was much more advantageous in terms of GSD and CMV yield than the use of sorbitol at 100 mg/mL, which was not able to efficiently preserve the initial CMV conditions. In fact, the CMV suspension containing sorbitol at about 100 mg/mL (S3A) was characterized by low CMV yield, with a maximum value of 6%, and by high GSD values, comprised between 1.27 and 1.38.

Moreover, these results confirmed that the CMV suspension containing PEG8000 at a concentration of about 200 mg/mL (2B) was characterized by a lower GSD value and a remarkable higher CMV yield when compared with the CMV suspension containing PEG8000 at about 100 mg/mL (2A). For instance, in the former case the CMV yield was found to be comprised between 56 and 68%, while in the latter case the higher CMV yield was found to be 45% at -40°C. Furthermore, also the GSD values were lower for the formulation 2B, with values lower than 1.20, confirming the higher performance of the solution of PEG8000 at 200mg/mL in preserving the initial characteristics of the CMV.

**Table 5** GSD value, CMV yield and concentration measured after freeze-drying (FD) at different freezing temperatures.

| Prep No. | FD protecting components (mg/mL) | Freezing T° (°C) | GSD | CMV Yield after FD (2-6 $\mu$m) (%) | Concentration (CMV/mL) |
|---|---|---|---|---|---|
| 2A | PEG8000 (100 mg/mL) | -60 °C | 1.21 | 26 | $0.9 \times 10^8$ |
| | | -50 °C | 1.21 | 39 | $1.3 \times 10^8$ |
| | | -40 °C | 1.22 | 45 | $2.1 \times 10^8$ |
| | | -30 °C | 1.21 | 34 | $1.5 \times 10^8$ |
| 2B | PEG8000 (200 mg/mL) | -60 °C | 1.20 | 64 | $2.0 \times 10^8$ |
| | | -50 °C | 1.20 | 68 | $2.0 \times 10^8$ |
| | | -40 °C | 1.19 | 61 | $2.3 \times 10^8$ |
| | | -30 °C | 1.20 | 56 | $2.5 \times 10^8$ |
| S3A | Sorbitol | -60 °C | 1.33 | 3 | $0.1 \times 10^8$ |
| | (100 mg/mL) | -50 °C | 1.29 | 3 | $0.1 \times 10^8$ |
| | | -40 °C | 1.38 | 6 | $0.3 \times 10^8$ |
| | | -30 °C | 1.27 | 4 | $0.2 \times 10^8$ |

*Example 5*

*Effect of the concentration of the freeze-drying protecting component on calibrated microvesicles characteristics*

The concentration of the freeze-drying protecting component was also investigated in order to evaluate the freeze-drying efficiency, in terms of preservation of GSD value and microvesicles yield after freeze-drying.

For this study, PEG4000 and PEG8000 were selected as freeze-drying protecting components, since they evolved as the most promising among those here investigated. Different solutions in a range of concentrations between 50 mg/mL and 250 mg/mL were prepared for each investigated formulation.

The calibrated microvesicles suspended in the different solutions of freeze-drying protecting components were then aliquoted in DIN8R glass vials (1.5 mL suspension/vial) and transferred in the freeze dryer.

Two different freezing temperatures were investigated. The vials were cooled at - 60°C and -40°C, and freeze-dried for approximately 1 hour under vacuum. At the end of the procedure, a freeze-dried composition was obtained as a white homogenous dry solid. The head space was then filled with pure $C_4F_{10}$.

Results

**Table 6** GSD and CMV yield values of the CMV suspensions containing different concentrations percentages of PEG4000, after freeze-drying.

| PEG4000 | Freezing at - 60°C | | Freezing at -40°C | |
|---|---|---|---|---|
| *mg/mL* | *GSD* | *CMV yield* (%) | *GSD* | *CMV yield* (%) |
| 50 | 1.230 | 10% | 1.224 | 19% |
| 100 | 1.224 | 35% | 1.204 | 38% |
| 150 | 1.227 | 59% | 1.193 | 46% |
| 200 | 1.246 | 65% | 1.191 | 57% |
| 250 | 1.197 | 71% | 1.198 | 52% |

**Table 7** GSD and CMV yield values of the CMV suspensions containing different concentrations percentages of PEG8000, freeze-drying.

| PEG8000 | Freezing at - 60 °C | | Freezing at -40 °C | |
|---|---|---|---|---|
| *mg/mL* | *GSD* | *CMV yield* (%) | *GSD* | *CMV yield* (%) |
| 50 | 1.259 | 4% | 1.243 | 9% |
| 100 | 1.188 | 29% | 1.213 | 24% |
| 150 | 1.182 | 56% | 1.202 | 49% |
| 200 | 1.188 | 63% | 1.193 | 60% |
| 250 | 1.195 | 60% | 1.192 | 58% |

Results showed that there is a linear relationship between the final microvesicles yield after freeze-drying and the total concentration of the freeze-drying protecting component. As displayed in **Figure 4,** an increasing improvement of the freeze-drying efficiency was obtained using concentrations of freeze-drying protecting component between 50 mg/mL and 200 mg/mL for both PEG4000 and PEG8000.

The above results show that an increase of the concentration of the PEG solution has in general a positive effect on the GSD values and/or CMV yields.

**Cited references**

**EP 3 990 032 B1**

1. WO2018/041906 A1 - BRACCO SUISSE SA

2. *PCT application number* PCT/EP2019/055325

3. US2017/080113 A1 - GE Healthcare

4. WO97/29782 A1 - NICOMED IMAGING A/S

5. WO2004/069284 A2 - BRACCO RESEARCH SA [CH]

**Claims**

1. A freeze-dried composition comprising (i) an amphiphilic material comprising a phospholipid and (ii) a polyethylene glycol (PEG) having a molecular weight comprised between 2000 and 10000 g/mol which, upon reconstitution with a pharmaceutically acceptable solution in the presence of a biocompatible gas, provides a suspension of calibrated gas-filled microvesicles, wherein said reconstituted suspension of microvesicles has a geometric standard deviation (GSD) value of at least 1.22 or lower and wherein said polyethylene glycol (PEG) has a concentration between 12 and 25% (w/v%).

2. The freeze-dried composition according to claim 1, wherein said polyethylene glycol (PEG) has a molecular weight comprised between 4000 and 8000 g/mol.

3. The freeze-dried composition according to claim 2, wherein said polyethylene glycol (PEG) is selected from a PEG having a molecular weight of 4000 g/mol ($\pm$ 10%) or a PEG having a molecular weight of 8000 g/mol ($\pm$ 10%).

4. The freeze-dried composition according to any of the preceding claims 1 to 3, wherein said reconstituted suspension of calibrated microvesicles is **characterized by** a concentration of at least $2.0 \times 10^8$ microvesicles/mL.

5. The freeze-dried composition according to any of the preceding claims 1 to 4, wherein said polyethylene glycol (PEG) has a concentration between 14 and 24% (w/v%).

6. A suspension of gas-filled microvesicles obtained by reconstituting a freeze-dried composition according to any of the preceding claims with a pharmaceutically acceptable solution in the presence of a biocompatible gas.

7. A suspension of gas-filled microvesicles according to claim 6 wherein the freeze-drying protecting component is present at a concentration between 12% and 25%.

8. A method of preparing a freeze-dried composition for the preparation of a reconstituted suspension of calibrated gas-filled microvesicles having a geometric standard deviation (GSD) of 1.22 or lower, comprising the steps of:

   a. preparing a suspension of calibrated gas-filled microvesicles comprising a polyglycol as freeze-drying protecting component, wherein said calibrated microvesicles have a size distribution **characterized by** a geometric standard deviation (GSD) of 1.22 or lower; and
   b. freeze-drying the calibrated microvesicles suspension.

9. The method of claim 8, wherein the method of preparation of step a. is the microfluidic flow-focusing technique.

10. The method of claim 9, wherein said freeze-drying protecting component has a total concentration comprised between 100 mg/ml and 300 mg/mL, preferably between 120 mg/ml and 250 mg/mL, more preferably the total concentration is 200 mg/mL.

11. A freeze-dried composition for preparing a suspension of calibrated gas-filled microvesicles having a geometric standard deviation (GSD) of 1.22 or lower, said freeze-dried composition being obtainable by a process comprising the following steps:

    a. preparing a first suspension of gas-filled calibrated microvesicles by a microfluidic flow-focusing process, said suspension further comprising a polyethylene glycol (PEG) having a molecular weight comprised between 2000 and 10000 g/mol and wherein said calibrated microvesicles have a size distribution **characterized by** a geometric standard deviation (GSD) of 1.22 or lower; and

b. freeze-drying said suspension.

12. A process for the preparation of an injectable contrast agent comprising a suspension of gas-filled microvesicles, wherein said process comprises reconstituting a freeze-dried composition as defined in any of the preceding claims 1-5 with a pharmaceutically acceptable solution in the presence of a biocompatible gas.

13. The process of claim 12, wherein said suspension of calibrated microvesicles is **characterized by** a GSD of 1.22 or lower.

**Patentansprüche**

1. Gefriergetrocknete Zusammensetzung umfassend (i) ein amphiphiles Material, das ein Phospholipid umfasst, und (ii) ein Polyethylenglycol (PEG) mit einem Molekulargewicht in dem Bereich zwischen 2000 und 10000 g/mol, die bei Rekonstitution mit einer pharmazeutisch annehmbaren Lösung in Gegenwart eines biokompatiblen Gases eine Suspension von kalibrierten gasgefüllten Mikrovesikeln bereitstellt, wobei die rekonstituierte Suspension von Mikrovesikeln einen Wert der geometrischen Standardabweichung (GSD) von wenigstens 1,22 oder darunter aufweist und wobei das Polyethylenglycol (PEG) eine Konzentration zwischen 12 und 25 % (Gew./Vol.-%) aufweist.

2. Gefriergetrocknete Zusammensetzung gemäß Anspruch 1, wobei das Polyethylenglycol (PEG) ein Molekulargewicht in dem Bereich zwischen 4000 und 8000 g/mol aufweist.

3. Gefriergetrocknete Zusammensetzung gemäß Anspruch 2, wobei das Polyethylenglycol (PEG) ausgewählt ist aus einem PEG mit einem Molekulargewicht von 4000 g/mol ($\pm$ 10 %) und einem PEG mit einem Molekulargewicht von 8000 g/mol ($\pm$ 10 %).

4. Gefriergetrocknete Zusammensetzung gemäß einem der vorstehenden Ansprüche 1 bis 3, wobei die rekonstituierte Suspension von kalibrierten Mikrovesikeln durch eine Konzentration von wenigstens $2,0 \times 10^8$ Mikrovesikeln/ml gekennzeichnet ist.

5. Gefriergetrocknete Zusammensetzung gemäß einem der vorstehenden Ansprüche 1 bis 4, wobei das Polyethylenglycol (PEG) eine Konzentration zwischen 14 und 24 % (Gew./Vol.-%) aufweist.

6. Suspension von gasgefüllten Mikrovesikeln, erhalten durch Rekonstituieren einer gefriergetrockneten Zusammensetzung gemäß einem der vorstehenden Ansprüche mit einer pharmazeutisch annehmbaren Lösung in Gegenwart eines biokompatiblen Gases.

7. Suspension von gasgefüllten Mikrovesikeln gemäß Anspruch 6, wobei die Gefriertrocknungs-Schutzkomponente in einer Konzentration zwischen 12 % und 25 % vorhanden ist.

8. Verfahren zur Herstellung einer gefriergetrockneten Zusammensetzung für die Herstellung einer rekonstituierten Suspension von kalibrierten gasgefüllten Mikrovesikeln mit einer geometrischen Standardabweichung (GSD) von 1,22 oder darunter, umfassend die Schritte:

   a. Herstellen einer Suspension von kalibrierten gasgefüllten Mikrovesikeln umfassend ein Polyglycol als Gefriertrocknungs-Schutzkomponente, wobei die kalibrierten Mikrovesikel eine Größenverteilung aufweisen, die durch eine geometrische Standardabweichung (GSD) von 1,22 oder darunter gekennzeichnet ist; und

   b. Gefriertrocknen der Suspension von kalibrierten Mikrovesikeln.

9. Verfahren gemäß Anspruch 8, wobei das Verfahren zur Herstellung Schritt a. das mikrofluidische flussfokussierende Verfahren ist.

10. Verfahren gemäß Anspruch 9, wobei die Gefriertrocknungs-Schutzkomponente eine Gesamtkonzentration in dem Bereich zwischen 100 mg/ml und 300 mg/ml aufweist, vorzugsweise zwischen 120 mg/ml und 250 mg/ml, wobei die Gesamtkonzentration bevorzugter 200 mg/ml beträgt.

**11.** Gefriergetrocknete Zusammensetzung für die Herstellung einer Suspension von kalibrierten gasgefüllten Mikrovesikeln mit einer geometrischen Standardabweichung (GSD) von 1,22 oder darunter, wobei die gefriergetrocknete Zusammensetzung durch ein Verfahren erhältlich ist, das die folgenden Schritte umfasst:

　　a. Herstellen einer ersten Suspension von gasgefüllten kalibrierten Mikrovesikeln durch ein mikrofluidisches flussfokussierendes Verfahren, wobei die Suspension ferner ein Polyethylenglycol (PEG) mit einem Molekulargewicht in dem Bereich zwischen 2000 und 10000 g/mol umfasst und wobei die kalibrierten Mikrovesikel eine Größenverteilung aufweisen, die durch eine geometrische Standardabweichung (GSD) von 1,22 oder darunter gekennzeichnet ist; und
　　b. Gefriertrocknen der Suspension.

**12.** Verfahren zur Herstellung eines injizierbaren Kontrastmittels, das eine Suspension von gasgefüllten Mikrovesikeln umfasst, wobei das Verfahren Rekonstituieren einer gefriergetrockneten Zusammensetzung gemäß einem der vorstehenden Ansprüche 1-5 mit einer pharmazeutisch annehmbaren Lösung in Gegenwart eines biokompatiblen Gases umfasst.

**13.** Verfahren gemäß Anspruch 12, wobei die Suspension von kalibrierten Mikrovesikeln durch eine GSD von 1,22 oder darunter gekennzeichnet ist.

## Revendications

**1.** Composition lyophilisée comprenant (i) une matière amphiphile comprenant un phospholipide et (ii) un polyéthylène glycol (PEG) ayant un poids moléculaire compris entre 2 000 et 10 000 g/mole qui, lors d'une reconstitution avec une solution pharmaceutiquement acceptable en la présence d'un gaz biocompatible, fournit une suspension de microvésicules remplies de gaz calibrées, ladite suspension reconstituée de microvésicules ayant une valeur d'écart type géométrique (GSD) d'au moins 1,22 ou moins et ledit polyéthylène glycol (PEG) ayant une concentration comprise entre 12 et 25 % (% p/v).

**2.** Composition lyophilisée selon la revendication 1, ledit polyéthylène glycol (PEG) ayant un poids moléculaire compris entre 4 000 et 8 000 g/mole.

**3.** Composition lyophilisée selon la revendication 2, ledit polyéthylène glycol (PEG) étant choisi parmi un PEG ayant un poids moléculaire de 4 000 g/mole (± 10 %) et un PEG ayant un poids moléculaire de 8 000 g/mole (± 10 %).

**4.** Composition lyophilisée selon l'une quelconque des revendications 1 à 3, ladite suspension reconstituée de microvésicules calibrées étant **caractérisée par** une concentration d'au moins $2,0 \times 10^8$ microvésicules/ml.

**5.** Composition lyophilisée selon l'une quelconque des revendications 1 à 4, ledit polyéthylène glycol (PEG) ayant une concentration comprise entre 14 et 24 % (% p/v).

**6.** Suspension de microvésicules remplies de gaz obtenue par reconstitution d'une composition lyophilisée selon l'une quelconque des revendications précédentes avec une solution pharmaceutiquement acceptable en la présence d'un gaz biocompatible.

**7.** Suspension de microvésicules remplies de gaz selon la revendication 6, le composant de protection de lyophilisation étant présent en une concentration comprise entre 12 % et 25 %.

**8.** Procédé de préparation d'une composition lyophilisée pour la préparation d'une suspension reconstituée de microvésicules remplies de gaz calibrées ayant un écart type géométrique (GSD) de 1,22 ou moins, comprenant les étapes de :

　　a. préparation d'une suspension de microvésicules remplies de gaz calibrées comprenant un polyglycol en tant que composant de protection de lyophilisation, lesdites microvésicules calibrées ayant une distribution de tailles **caractérisée par** un écart type géométrique (GSD) de 1,22 ou moins ; et
　　b. lyophilisation de la suspension de microvésicules calibrées.

**9.** Procédé selon la revendication 8, le procédé de préparation de l'étape a. étant la technique de focalisation de flux microfluidique.

10. Procédé selon la revendication 9, ledit composant de protection de lyophilisation ayant une concentration totale comprise entre 100 mg/ml et 300 mg/ml, préférablement entre 120 mg/ml et 250 mg/ml, plus préférablement la concentration totale étant de 200 mg/ml.

11. Composition lyophilisée pour la préparation d'une suspension de microvésicules remplies de gaz calibrées ayant un écart type géométrique (GSD) de 1,22 ou moins, ladite composition lyophilisée pouvant être obtenue par un processus comprenant les étapes suivantes :

a. préparation d'une première suspension de microvésicules calibrées remplies de gaz par un processus de focalisation de flux microfluidique, ladite suspension comprenant en outre un polyéthylène glycol (PEG) ayant un poids moléculaire compris entre 2 000 et 10 000 g/mole et lesdites microvésicules calibrées ayant une distribution de tailles **caractérisée par** un écart type géométrique (GSD) de 1,22 ou moins ; et
b. lyophilisation de ladite suspension.

12. Processus pour la préparation d'un agent de contraste injectable comprenant une suspension de microvésicules remplies de gaz, ledit processus comprenant une reconstitution d'une composition lyophilisée telle que définie dans l'une quelconque des revendications précédentes 1 à 5 avec une solution pharmaceutiquement acceptable en la présence d'un gaz biocompatible.

13. Processus selon la revendication 12, ladite suspension de microvésicules calibrées étant **caractérisée par** un GSD de 1,22 ou moins.

*Figure 1*

*Figure 2*

*Figure 3*

*Figure 4*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018041906 A1
- EP 2019055325 W
- US 2017080113 A1
- WO 9729782 A1
- WO 2004069284 A2